# EUROPEAN PATENT APPLICATION

(11) **EP 2 417 963 A1**
(43) Date of publication of application: **15.02.2012**
(21) Application number: 10761639.3
(22) Date of filing: 31.03.2010
(51) Int. Cl.: A61K 8/41, A61K 8/34, A61K 8/36, A61K 8/368, A61K 8/46, A61Q 5/12

(54) **HAIR COSMETIC**

(30) Priority: 10.04.2009 JP 2009095423
(71) Applicant: Shiseido Company, Ltd., Chuo-ku Tokyo 104-8010 (JP)
(72) Inventor: NAGANO Tanemasa, Yokohama-shi Kanagawa 224-8558 (JP); WATANABE Tomoko, Yokohama-shi Kanagawa 224-8558 (JP); IWAI Shigeru, Yokohama-shi Kanagawa 224-8558 (JP)
(74) Representative: Schön, Christoph
(86) International application number: PCT/JP2010/055969
(87) International publication number: WO 2010/116940

(57) **Abstract**

To provide means for improving sensation of hair in application of a hair cosmetic composition, particularly a hair conditioner, after washing of the hair. The present inventors have found that the object can be attained by a hair cosmetic composition containing the following ingredients (1) to (4):
(1) a hydroxyetheramine compound represented by formula (I) in an amount of 0.01 to 10 mass% with respect to the hair cosmetic composition: 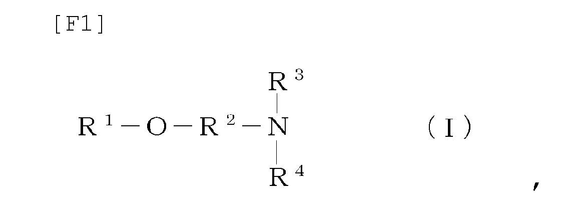 wherein R¹ represents a C6 to C24 linear or branched alkyl group or alkenyl group, R² represents a C2 to C6 linear or branched hydroxyalkylene or hydroxyalkylenyl group, and R³ and R⁴, which are identical to or different from each other, each represent a hydrogen atom or a C1 to C6 linear alkyl group;
(2) a higher alcohol and/or a higher fatty acid in an amount of 0.1 to 20 mass% with respect to the hair cosmetic composition;
(3) an organic acid having an aromatic ring skeleton; and
(4) water.

## Description

### Technical Field

The present invention relates to a hair cosmetic composition.

### Background Art

Generally, after hair washing (e.g., shampooing), the gloss of the hair tends to be reduced, and fingers do not smoothly run through the hair. To prevent this, a hair-treating agent such as a hair conditioner is used.

The hair-treating agent generally contains, as an additive, a quaternary ammonium salt cationic surfactant. Since the quaternary ammonium salt cationic surfactant effectively imparts softness and anti-static property to the hair, the surfactant is incorporated into hair-treating agents as well as into various hair cosmetic compositions.

For attaining a satisfactory hair conditioning effect, in particular, imparting favorable smoothness and moistness to dried hair, there have been provided a hair cosmetic composition containing a hydroxyetheramine compound instead of a quaternary ammonium salt cationic surfactant (see Patent Documents 1 and 2) and a hair cosmetic composition containing an amidoamine instead of a quaternary ammonium salt cationic surfactant (Patent Document 3).

Also disclosed are a hair cosmetic composition containing alkoxypropyldimethylamine (Patent Documents 4 and 5), and a hair cosmetic composition containing high-viscosity dimethylpolysiloxane for improving tactile sensation during use (Patent Document 6).

### Prior Art Documents

### Patent Documents

Patent Document 1: Japanese Patent Application Laid-Open *(kokai)* No. 2004-323495
Patent Document 2: Japanese Patent Application Laid-Open *(kokai)* No. 2007-161605
Patent Document 3: Japanese Patent Application Laid-Open *(kokai)* No. Hei 9-71516
Patent Document 4: Japanese Patent Application Laid-Open *(kokai)* No. 2004-67534
Patent Document 5: Japanese Patent Application Laid-Open *(kokai)* No. 2004-2261
Patent Document 6: Japanese Patent Application Laid-Open *(kokai)* No. Hei 4-305516

### Summary of the Invention

### Problems to be Solved by the Invention

An object of the present invention is to provide means for improving tactile sensation of hair during application of a hair cosmetic composition, particularly a hair conditioner (hereinafter may be also referred to as tactile sensation upon application or application sensation), after washing of the hair.

### Means for Solving the Problems

The present inventors have carried out extensive studies so as to attain the aforementioned object and, surprisingly, have found that a hair cosmetic composition having the following formulation can attain the aforementioned object.

Accordingly, the present invention provides a hair cosmetic composition comprising the following ingredients (1) to (4) (hereinafter may be also referred to as the hair cosmetic composition of the present invention):
(1) a hydroxyetheramine compound represented by formula (I) in an amount of 0.01 to 10 mass% with respect to the hair cosmetic composition:

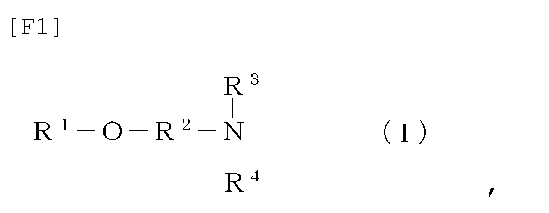

wherein R¹ represents a C6 to C24 linear or branched alkyl group or alkenyl group, R² represents a C2 to C6 linear or branched hydroxyalkylene or hydroxyalkylenyl group, and R³ and R⁴, which are identical to or different from each other, each represent a hydrogen atom or a C1 to C6 linear alkyl group;
(2) a higher alcohol and/or a higher fatty acid in an amount of 0.1 to 20 mass% with respect to the hair cosmetic composition;
(3) an organic acid having an aromatic ring skeleton; and
(4) water.

The aforementioned hydroxyetheramine compound (I) is preferably N-(2-hydroxy-3-stearoxypropyl)-N,N-dimethylamine, represented by (II):

CH₃(CH₂)₁₆CH₂-O-CH₂CH(OH)CH₂-N(CH₃)₂ (II) .

### Effects of the Invention

The present invention enables provision of a hair cosmetic composition which provides favorable hair sensation upon application of the hair cosmetic composition (e.g., hair conditioner) after washing the hair.

### Modes for Carrying Out the Invention

### <Essential ingredients>

The hair cosmetic composition of the present invention contains the aforementioned essential ingredients (1) to (4).

### (1) Hydroxyetheramine compound (I)

Examples of the C6 to C24 linear or branched alkyl or alkenyl group of R¹ include hexyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, myristoleyl, palmitoleyl, oleyl, linolyl, linoleyl, ricinoleyl, and isostearyl. Examples of the C2 to C6 linear or branched divalent group; i.e., hydroxyalkylene or hydroxyalkylenyl group of R² include hydroxyethylene, hydroxytrimethylene, hydroxytetramethylene, hydroxypentamethylene, and hydroxyhexamethylene. As described above, R³ and R⁴, which are identical to or different from each other, each represent a hydrogen atom or a C1 to C6 linear alkyl group.

In the present invention, the hydroxyetheramine compound (I) is particularly preferably that in which R¹ is a stearyl group (octadecyl group), R² is -CH₂-CH(OH)CH₂-, and each of R³ and R⁴ is a methyl group, that is, N-(2-hydroxy-3-stearoxypropyl)-N,N-dimethylamine(II), as described above.

The hydroxyetheramine compound (I) may be produced through a customary method suited for its chemical structure. Specifically, N-(2-hydroxy-3-stearoxypropyl)-N,N-dimethylamine (II) may be produced through a customary method suited for its chemical structure, for example, a method disclosed in Patent Document 1, or any of other known methods.

Briefly, a hydroxyetheramine compound (I) of interest may be produced through the following procedure. Firstly, a higher alcohol and a BF₃-ether complex are fed to a reactor. While the mixture is heated at 50 to 90°C under stirring, epichlorohydrin is added dropwise to the reactor. The reaction system is aged, to thereby form 1-chloro-3-alkoxy-2-hydroxypropane. The thus-obtained 1-chloro-3-alkoxy-2-hydroxypropane is aged with 25% NaOH, to thereby form aqueous and oil layers. The aqueous layer is removed therefrom, and the oil layer is dehydrated, to thereby yield 1,2-epoxy-3-alkoxypropane. To the thus-formed 1,2-epoxy-3-alkoxypropane, dimethylamine or monomethylamine is added over 2 to 3 hours. The reaction mixture is aged, and then excessive dimethylamine or monomethylamine and the like are distilled away under reduced pressure, to thereby yield the target hydroxyetheramine compound (I).

In the hair cosmetic composition of the present invention, the amount of hydroxyetheramine compound (I) incorporated into the hair cosmetic composition is 0.01 to 10 mass% with respect to the total amount of hair cosmetic composition, preferably 0.1 to 5 mass%. When the hydroxyetheramine content is less than 0.01 mass%, enhancement in tactile sensation upon application of the composition of the invention is not virtually expected, and the hair-conditioning effect by virtue of incorporation of the hydroxyetheramine compound (I) is likely to be reduced, whereas when the hydroxyetheramine content is in excess of 10 mass%, no substantial application-sensation-improving effect commensurate with addition in large amount is attained.

### (2) Higher alcohol and higher fatty acid

To the hair cosmetic composition of the present invention, one or more members selected from among higher alcohols or higher fatty acids which are employed in external use compositions such as cosmetics may be added. The higher alcohol and higher fatty acid may be used in combination.

Examples of the higher alcohol include lauryl alcohol, myristyl alcohol, cetyl alcohol, cetostearyl alcohol, stearyl alcohol, arachyl alcohol, behenyl alcohol, oleyl alcohol, jojoba alcohol, chimyl alcohol, and batyl alcohol. Examples of the higher fatty acid include lauric acid, myristic acid, palmitic acid, stearic acid, behenic acid, oleic acid, linoleic acid, undecylenic acid, 12-hydroxystearic acid, lanolin fatty acid, and isostearic acid. Among them, stearyl alcohol is preferred as a higher alcohol, and stearic acid is preferred as a higher fatty acid.

The hair cosmetic composition of the present invention contains the higher fatty acid and/or higher alcohol in an amount of 0.1 to 20 mass% with respect to the total amount of hair cosmetic composition, preferably 1 to 10 mass%. When the higher fatty acid and/or higher alcohol content is less than 0.1 mass%, enhancement in tactile sensation upon application of the composition of the invention is not virtually expected, and the hair-conditioning effect by virtue of incorporation of the higher fatty acid and/or higher alcohol is likely to be reduced, whereas when the higher fatty acid and/or higher alcohol content is in excess of 20 mass%, no substantial application-sensation-improving effect commensurate with addition in large amount is attained.

### (3) Organic acid having an aromatic ring skeleton

Examples of preferred organic acids having an aromatic ring skeleton (hereinafter may be also referred to as "aromatic organic acids") which may be incorporated into the hair cosmetic composition of the present invention include salicylic acids (e.g., salicylic acid, 3-methylsalicylic acid, 4-methylsalicylic acid, and 5-methylsalicylic acid), toluenesulfonic acid (e.g., p-toluenesulfonic acid, o-toluenesulfonic acid, and m-toluenesulfonic acid), benzoic acids (e.g., benzoic acid, 2-methylbenzoic acid, 3-methylbenzoic acid, and 4-methylbenzoic acid), and naphthalenesulfonic acid salts (e.g., 2-naphthalenesulfonic acid salt). These aromatic organic acids may be used singly or in combination of two or more species in accordance with needs. Among them, salicylic acids are particularly preferred.

No particular limitation is imposed on the amount of aromatic organic acid incorporated into the hair cosmetic composition of the present invention, and the amount is preferably 0.1 mol or more with respect to the aforementioned hydroxyetheramine compound (I), more preferably 0.5 to 1 mol. When the amount is less than 0.1 mol, tactile sensation upon application of the composition is likely to be impaired as the viscosity of the composition decreases. Although no particular limitation is imposed on the upper limit of the amount, when the amount is in excess of 1 mol, difficulty is encountered in attaining application-sensation-improving effect commensurate with the amount of addition, and odor, irritation to the skin, or the like caused by an excess amount of aromatic organic acid tends to be provided.

### (4) Water

Water employable in the hair cosmetic composition may be any of ion-exchanged water, purified water, tap water, and natural water, and the type and amount of water may be selected in accordance with the form and product type of the hair cosmetic composition of the present invention. Specifically, in a typical manner, the hair cosmetic composition is formed from the aforementioned essential ingredients (mass%), other ingredients selected in consideration of the form and product type of the hair cosmetic composition (mass%), and water as balance.

### <Optional ingredients incorporated into the hair cosmetic composition>

### (1) Alcohol having an aromatic ring skeleton

Through incorporation of an alcohol having an aromatic ring skeleton into the hair cosmetic composition of the present invention, tactile sensation upon application of the composition can be further improved. Examples of the alcohol having an aromatic ring skeleton include phenoxyethanol and benzyl alcohol. Of these, phenoxyethanol is preferred. The amount of alcohol having an aromatic ring skeleton incorporated into the hair cosmetic composition of the present invention is 0.01 to 3 mass% with respect to the cosmetic composition, preferably 0.1 to 1 mass%. When the amount is less than 0.01 mass%, further enhancement in tactile sensation upon application of the composition of the invention is not virtually expected, and difficulty is encountered in further enhancement of the hair-conditioning effect by virtue of incorporation of the higher fatty acid and/or higher alcohol. When the amount is in excess of 3 mass%, additional enhancement in tactile sensation upon application of the cosmetic composition reaches the plateau, and no substantial application-sensation-improving effect commensurate with the amount of addition is attained.

### (2) Other ingredients

If required, the hair cosmetic composition of the present invention may further contain a certain additional ingredient other than the aforementioned ingredients in such qualitative and quantitative range that the effects of the present invention are not substantially impaired. For example, in the case where the hair cosmetic composition of the present invention is a hair conditioner or a hair rinse, a cationic surfactant such as alkyltrimethylammonium chloride is generally employed as an additional ingredient. In the case where the hair cosmetic composition of the present invention is a shampoo, typical examples of the additional ingredient employed include anionic surfactants such as alkylsulfate ester salts, polyoxyethylene alkyl ether sulfate ester salts, acylmethyltauric acid, and N-acylglutamic acid salts; amphoteric surfactants such as alkylbetaine, alkylamidobetaine, and imidazoliniumbetaine; and nonionic surfactants such as fatty acid alkanolamines.

Regardless of the form thereof, the hair cosmetic composition may further contain other additives. Specific examples include oily ingredients (other than the aforementioned higher fatty acids and higher alcohols) such as hydrocarbon oils, ester oils, and silicone oils; humectants such as glycerin, propylene glycol, 1,3-butylene glycol, and polyethylene glycol; conditioning agents such as cationic polymer (e.g., cationized cellulose); anti-dandruff agents such as trichlorocarbanilide, sulfur, zinc pyrithione, and isopropylmethyl phenol; a thickener; a viscosity-controlling agent; an emulsifying agent; a sequestering agent; a UV-absorber; an anti-oxidant; an antiseptic agent; powder ingredients; hair-growing agents such as a blood flow-promoting agent, a local stimulant, a hair follicle-activating agent, an anti-androgenic agent, an anti-seborrheic agent, a keratolytic agent, a bactericide, an anti-inflammatory agent, amino acid, vitamins, and herbal medicines; a pH-regulating agent; a pigment; perfume; and lower alcohols.

### (3) The hair cosmetic composition of the present invention

The hair cosmetic composition of the present invention may be produced through a suitable method selected in accordance with the form and product type of the composition. In one typical method, the aforementioned essential ingredients and optional ingredients are dissolved in a solvent such as water. Examples of the product type include hair rinse, hair conditioner, hair treatment, hair shampoo, and rinse-in-shampoo.

### Examples

The present invention will next be described in detail by way of examples, which should not be construed as limiting the invention thereto. Unless otherwise specified, the amount of each ingredient is based on mass%.

### <Method of evaluation>

The hair cosmetic composition of the present invention was evaluated as follows:

### (1) Test for tactile sensation during use

Tactile sensation upon actual application of a hair cosmetic composition was tested with six female panelists. Specifically, using hands, each panelist uniformly applied an appropriate amount of a test cosmetic sample (hair rinse) to the hair in a wet state immediately after hair washing, and the tactile sensation upon application was evaluated by the panelist in terms of (1) spreadability of the cosmetic composition on the hair, (2) suppleness of the hair, (3) smoothness of the hair, and (4) moistness of the hair, based on the following ratings.

○○: Four or more of the six panelists answered "good."
○: Three of the six panelists answered "good."
○△: Two of the six panelists answered "good."
△: One of the six panelists answered "good."
X: None of the six panelists answered "good."

### <Test Example>

Hair rinse products having formulations shown in Table 1 were prepared by dissolving the ingredients in purified water with mixing. The thus-prepared hair rinse products were subjected to the above test. The results are also shown in Table 1. In Table 1, "stearoxyhydroxypropylamine" means "N-(2-hydroxy-3-stearoxypropyl)-N,N-dimethylamine (II)."

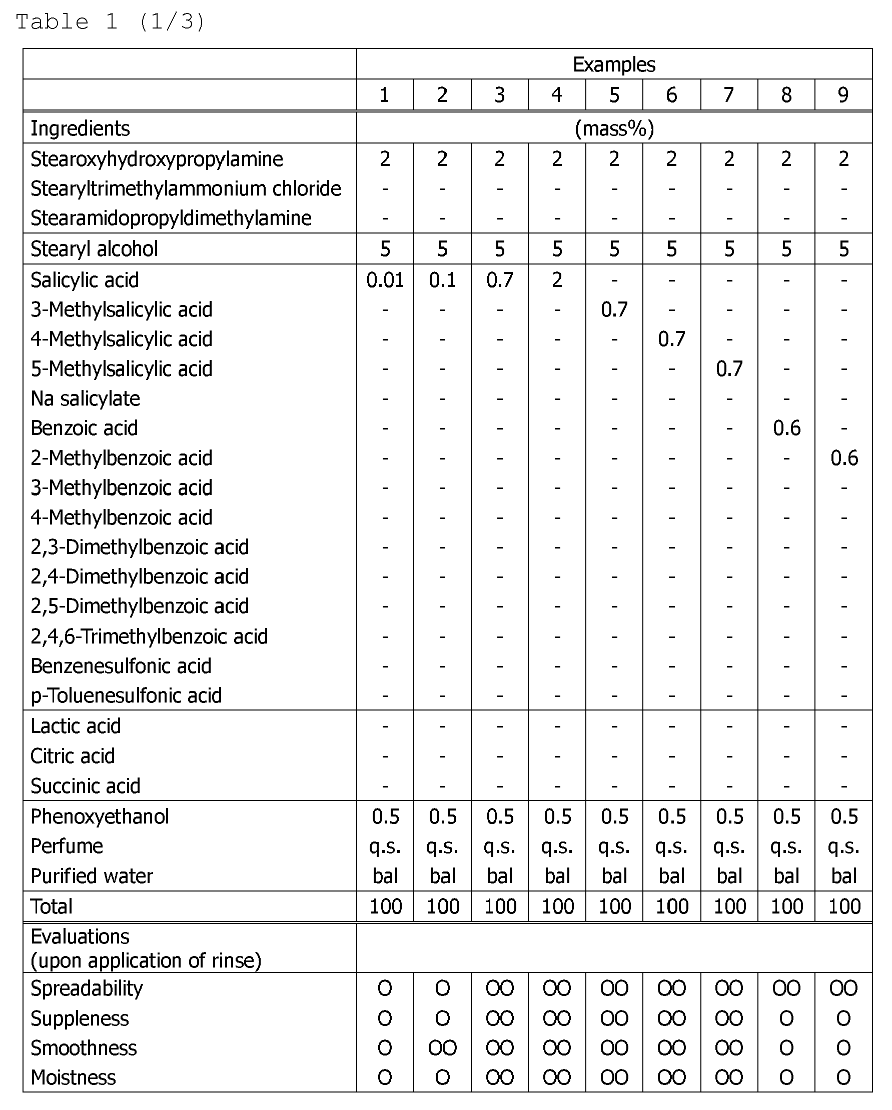

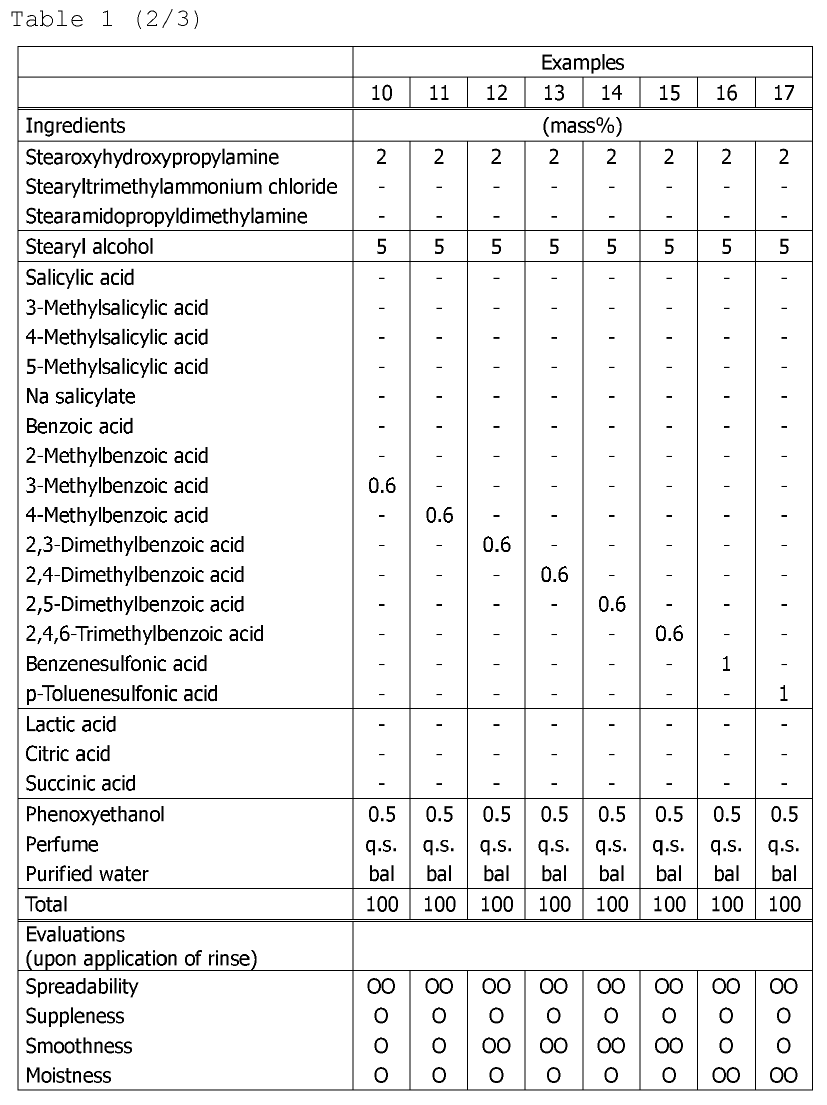

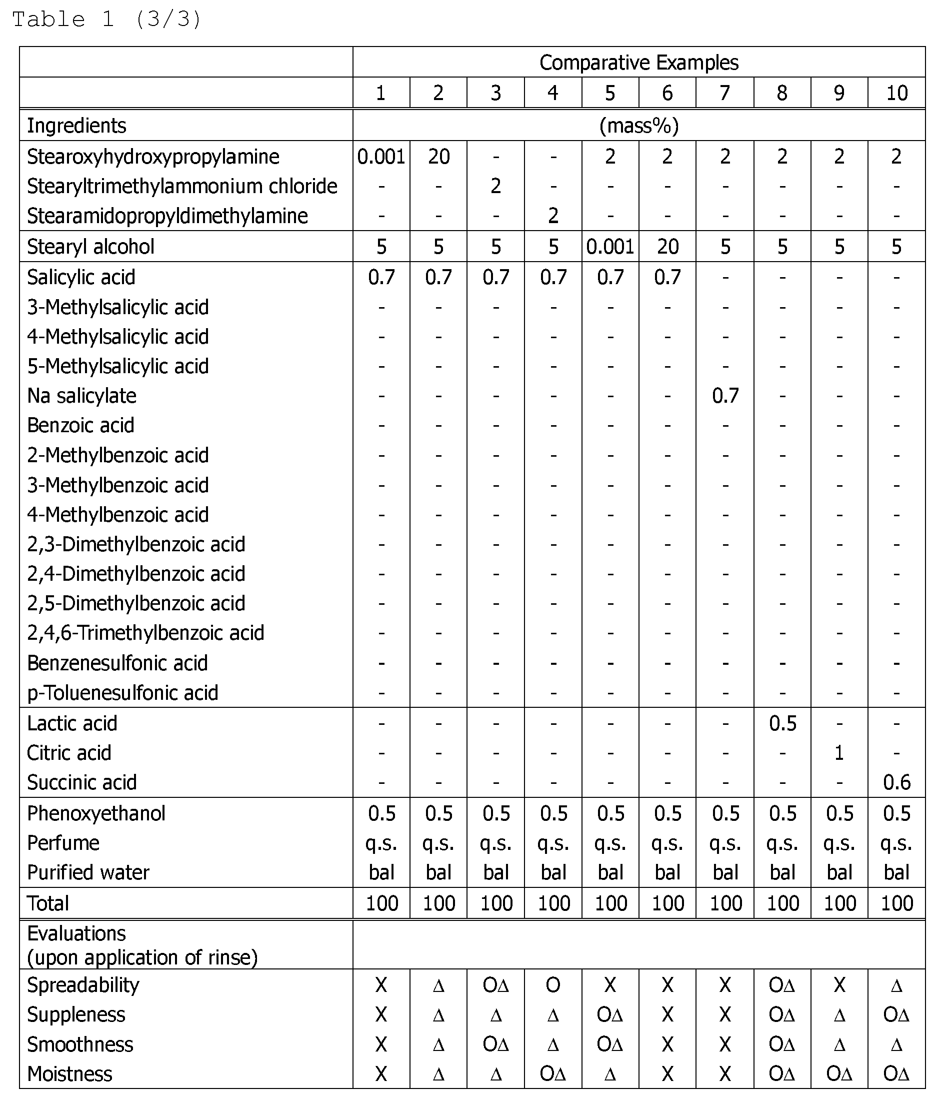

As is clear from Table 1, in Examples 1 to 17, which falls within the scope of the present invention, all the tested aromatic organic acids were found to improve tactile sensation upon application of the hair rinse products, which is the target effect of the present invention. In Comparative Example 1, in which the amount of N-(2-hydroxy-3-stearoxypropyl)-N,N-dimethylamine (II) (hydroxyetheramine compound (I)) was small but the amounts of the other ingredients fell within the scope of the present invention, tactile sensation upon application of the hair rinse product was poor for all the evaluation items. In Comparative Example 2, in which the amount of N-(2-hydroxy-3-stearoxypropyl)-N,N-dimethylamine (II) incorporated into the hair rinse product was in excess of the upper limit of the present invention, tactile sensation upon application of the hair rinse product was also poor. In Comparative Examples 3 and 4, in which stearyltrimethylammonium chloride or steramidopropyldimethylamine (quaternary ammonium) was used instead of N-(2-hydroxy-3-stearoxypropyl)-N,N-dimethylamine (II), tactile sensation upon application of each hair rinse product was inferior to that attained by the hair cosmetic composition of the present invention.

In Comparative Example 5, in which the amount of stearyl alcohol (higher alcohol) was small (outside the scope of the invention), tactile sensation upon application of the hair rinse product was inferior to that attained by the hair cosmetic composition of the present invention. In Comparative Example 6, in which the amount of stearyl alcohol was in excess of the upper limit of the present invention, tactile sensation upon application of the hair rinse product was poor.

In Comparative Example 7, in which sodium salicylate (aromatic organic acid salt) was used instead of salicylic acid (aromatic organic acid), tactile sensation upon application of the hair rinse product was poor.

In Comparative Examples 8 to 10, in which an organic acid having no aromatic ring was used instead of aromatic organic acid, tactile sensation upon application of each hair rinse product was inferior to that attained by the hair cosmetic composition of the present invention.

The meritorious effect of phenoxyethanol-an alcohol having an aromatic ring skeleton-was confirmed in the Examples but not confirmed in the Comparative Examples.

Formulation examples of the hair cosmetic composition of the present invention will next be given.

### Formulation Example 1: Hair conditioner

| | Formulation | Amount (mass%) |
|---|---|---|
| (1) | N-(2-Hydroxy-3-stearoxypropyl)-N,N-dimethylamine | 1.5 |
| (2) | Taurine | 1.0 |
| (3) | Salicylic acid | 0.5 |
| (4) | Stearyl alcohol | 5.0 |
| (5) | Stearic acid | 0.5 |
| (6) | Glyceryl monostearate | 0.5 |
| (7) | Pyrrolidonecarboxylic acid | 0.4 |
| (8) | Hydroxyethylurea | 0.4 |
| (9) | Camellia reticulata seed oil | 0.1 |
| (10) | High polymerized polyethylene glycol | 0.1 |
| | (Mw: 4,000,000) | |
| (11) | Quaternary ammonium salt | 0.1 |
| | (Polyquaternium-61) | |
| (12) | Dimethicone (20 mPa·s) | 5.0 |
| (13) | Dimethiconol (10,000 mPa·s) | 1.0 |
| (14) | Mineral oil | 0.3 |
| (15) | Octyl palmitate | 0.3 |
| (16) | Sorbitol | 10.0 |
| (17) | Diglycerin | 3.0 |
| (18) | Isoprene glycol | 4.0 |
| (19) | POE(10) POP(7) dimethyl ether | 0.2 |
| | (random copolymer) | |
| (20) | Perfume | 0.6 |
| (21) | Cationized starch | 0.1 |
| (22) | Capsicum frutescens extract | 0.05 |
| (23) | Menthol | 0.1 |
| (24) | Vanillyl butyl ether | 0.02 |
| (25) | Oat extract | 0.1 |
| (26) | Phenoxyethanol | 0.4 |
| (27) | Benzyloxyethanol | 0.3 |
| (28) | Purified water | balance |

### Production method

The above ingredients other than purified water were heated to 70°C, to thereby melt solid contents. Water heated at 70°C was added to the above mixture under mixing and stirring. The resultant mixture was cooled to ambient temperature.

### Formulation Example 2: Hair conditioner

| | Formulation | Amount (mass%) |
|---|---|---|
| (1) | N-(2-Hydroxy-3-stearoxypropyl)-N,N-dimethylamine | 2.5 |
| (2) | Stearyl alcohol | 6.0 |
| (3) | Solid paraffin | 0.5 |
| (4) | Glyceryl monooleate | 0.4 |
| (5) | p-Toluenesulfonic acid | 0.6 |
| (7) | Perfume | 0.4 |
| (8) | L-Arginine | 0.2 |
| (9) | Dimethicone (1,000 mPa·s) | 0.5 |
| (10) | Amodimethicone (1,000 mPa·s) | 1.0 |
| (11) | Dimethiconol (4,000 mPa·s) | 2.0 |
| (12) | Isocetyl isostearate | 1.0 |
| (13) | Glycerin | 5.0 |
| (14) | Isoprene glycol | 2.0 |
| (15) | POE(35) POP(40) dimethyl ether (block copolymer) | 0.3 |
| (16) | Cationized cellulose | 0.5 |
| (17) | Loquat leaf extract | 0.2 |
| (18) | Methylparaben | 0.3 |
| (19) | Benzyl alcohol | 0.3 |
| (20) | Purified water | balance |

### Production method

The above ingredients other than purified water were heated to 80°C, to thereby melt solid contents. Water heated at 80°C was added to the above mixture under mixing and stirring. The resultant mixture was cooled to ambient temperature.

### Formulation Example 3: Hair treatment

| | Formulation | Amount (mass%) |
|---|---|---|
| (1) | N-(2-Hydroxy-3-stearoxypropyl)-N,N-dimethylamine | 1.0 |
| (2) | Phenoxyethanol | 0.8 |
| (3) | Stearyl alcohol | 5.0 |
| (4) | Stearic acid | 0.3 |
| (5) | 2-Naphthalenesulfonic acid | 0.5 |
| (6) | Pyrrolidonecarboxylic acid | 0.05 |
| (7) | Perfume | 0.4 |
| (8) | Dimethicone (6 mPa·s) | 10.0 |
| (9) | High polymerized methylpolysiloxane (1,000,000 mPa·s) | 1.0 |
| (10) | Amodimethicone (1,000 mPa·s) | 2.0 |
| (11) | Octyl palmitate | 1.0 |
| (12) | POE(5) isostearyl glyceryl ether | 0.2 |
| (13) | Sorbitol | 15.0 |
| (14) | Diglycerin | 2.0 |
| (15) | Dipropylene glycol | 10.0 |
| (16) | High polymerized polyethylene glycol (Mw: 2,000,000) | 0.2 |
| (17) | Hydroxyethylcellulose | 0.2 |
| (18) | Menthol | 0.1 |
| (19) | Rose extract | 0.4 |
| (20) | Soybean lecithin | 0.2 |
| (21) | Purified water | balance |

### Production method

The above ingredients other than purified water were heated to 70°C, to thereby melt solid contents. Water heated at 70°C was added to the above mixture under mixing and stirring. The resultant mixture was cooled to ambient temperature.

## Claims

1. A hair cosmetic composition comprising the following ingredients (1) to (4):
(1) a hydroxyetheramine compound represented by formula (I) in an amount of 0.01 to 10 mass% with respect to the hair cosmetic composition: 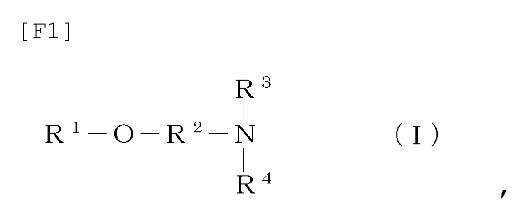 wherein R¹ represents a C6 to C24 linear or branched alkyl group or alkenyl group, R² represents a C2 to C6 linear or branched hydroxyalkylene or hydroxyalkylenyl group, and R³ and R⁴, which are identical to or different from each other, each represent a hydrogen atom or a C1 to C6 linear alkyl group;
(2) a higher alcohol and/or a higher fatty acid in an amount of 0.1 to 20 mass% with respect to the hair cosmetic composition;
(3) an organic acid having an aromatic ring skeleton; and
(4) water.

2. The hair cosmetic composition according to claim 1, wherein the hydroxyetheramine compound (I) is N-(2-hydroxy-3-stearoxypropyl)-N,N-dimethylamine, represented by formula (II) :
CH₃(CH₂)₁₆CH₂-O-CH₂CH(OH)CH₂-N(CH₃)₂ (II).

3. The hair cosmetic composition according to claim 1, wherein the organic acid having an aromatic ring skeleton is a salicylic acid, toluenesulfonic acid, a benzoic acid, or naphthalenesulfonic acid.

4. The hair cosmetic composition according to claim 2, wherein the organic acid having an aromatic ring skeleton is a salicylic acid, toluenesulfonic acid, a benzoic acid, or naphthalenesulfonic acid.
